(19) Europäisches Patentamt
European Patent Office
Office européen des brevets

(11) Numéro de publication : **0 080 419**
**B1**

(12) # FASCICULE DE BREVET EUROPÉEN

(45) Date de publication du fascicule du brevet : **24.01.90**

(51) Int. Cl.$^5$ : **C 07 D311/30**, C 07 D405/04, C 07 D407/04, C 07 D409/04

(21) Numéro de dépôt : **82402136.4**

(22) Date de dépôt : **24.11.82**

(54) **Haloalkyl-8-4H-(1)-benzopyran-4-ones, et procédés de préparation.**

(30) Priorité : **25.11.81 FR 8122019**

(43) Date de publication de la demande : **01.06.83 Bulletin 83/22**

(45) Mention de la délivrance du brevet : **24.01.90 Bulletin 90/04**

(84) Etats contractants désignés : **AT BE CH DE FR GB IT LI LU NL SE**

(56) Documents cités :
**EP-A- 0 080 394**
**DE-B- 1 270 567**
**FR-A- 2 291 745**
**FR-A- 2 326 919**
**US-A- 3 966 770**
**CHEMICAL ABSTRACTS, vol.83, 1975, page 471, résumé no. 27806t COLUMBUS OHIO (US)**
**JOURNAL OF ORGANIC CHEMISTRY, vol. 45, 1980 American Chemical Society B.J. WHITLOCK et al.:" Regiospecific Synthesis of Islandicin Methyl Ether", pages 12-15**
**BULLETIN DE LA SOCIETE CHIMIQUE DE FRANCE, no.9, 1966 G.REGNIER et al.: "No 473. -Acide-Phénols. I. - Sur la synthèse de dérivés pipéraziniques de l'acide salicylique.", pages 2821-7**

(73) Titulaire : **LIPHA, LYONNAISE INDUSTRIELLE PHARMACEUTIQUE**
**34, rue Saint Romain Boîte Postale 8481**
**F-69359 Lyon Cedex 08 (FR)**

(72) Inventeur : **Briet, Philippe**
**216, avenue Félix Faure**
**F-69003 Lyon (FR)**
Inventeur : **Berthelon, Jean-Jacques**
**1, allée des Glycines**
**F-69150 Decines Charpieu (FR)**
Inventeur : **Collonges, François**
**Le Grand Champ Chemin de Halage**
**F-01700 Beynost Miribel (FR)**

(74) Mandataire : **Sadones Laurent, Renée et al**
**L'AIR LIQUIDE 75, quai d'Orsay**
**F-75321 Paris Cedex 07 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

## Description

La présente invention concerne des alkyl-8-4H-[1]-benzopyran-4-ones, leur préparation, et les nouveaux intermédiaires utilisables dans celle-ci.

Les alkyl-8-4H-[1]-benzopyran-4-ones sont représentés par la formule

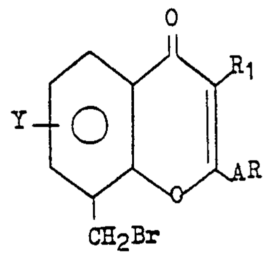

(I)

dans laquelle AR est radical méthyle, phényle, méthylphényle, méthoxyphényle, diméthoxyphényle, thényle, furyle, naphtyle, cyclohexyle ou benzyle, Y est l'hydrogène, $R_1$ est l'hydrogène.

Ils sont également représentés par la dite formule I dans laquelle AR est un radical phényle, $R_1$ est l'hydrogène, Y est un radical méthyle ou méthoxy.

Ils sont aussi représentés par la même formule I dans laquelle AR est l'hydrogène ou un radical phényle, $R_1$ est un radical phényle, Y est l'hydrogène.

Ces composés sont utilisables en tant que produits industriels intermédiaires de synthèse, en particulier dans le domaine des pesticides et des produits pharmaceutiques, tels les acides [oxo-4-4H-[1]-benzopyran-8-yl] alcanoïques et leurs dérivés faisant l'objet de la demande EP.80.934. Les bromoalkyl-8-4H-[1]-benzopyran-4-ones constituent une classe particulièrement intéressante.

Les bromobenzopyranones répondant à la formule I peuvent être préparées, avec de bons rendements, par cyclisation des propanediones-1,3 de formule

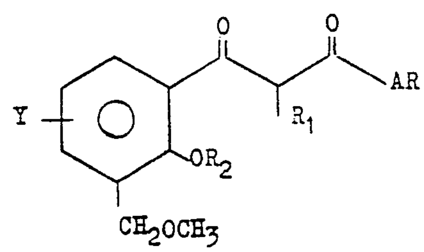

(II)

dans laquelle AR, $R_1$ et Y ont les mêmes significations que précédemment, $R_2$ est l'hydrogène ou un radical méthyle, en présence d'acide bromhydrique.

Il est donné ci-après des exemples qui illustrent l'invention à titre non limitatif.

### Exemple 1

Bromométhyl-8-phényl-2-phényl-2-4H-[1]-benzopyran-4-one (formule 1) $C_{16}H_{11}BrO_2$ · PM = 315,17.

On chauffe un mélange de 361 g (1,40 mole) d'(hydroxy-2-méthoxy-méthyl-3-phényl)-1-phényl-3-propanedione-1,3, 1,4 l d'acide acétique et 917 ml d'acide bromhydrique à 62 % pendant 3 heures à 70 °C. On verse ensuite dans 5 l d'eau froide, on filtre, on lave à l'eau et on recristallise dans l'acétone. On obtient 207,4 g (Rdt : 47 %) d'un solide blanc. $PF_G$ : 182-183 °C. IR : $\mu_c = 0$ : 1 650 $cm^{-1}$. RMN (DMSO) δ en ppm par rapport au TMS, 2 H à 5,02 (singulet), 1 H à 7,02 (singulet), 8 H de 7,3 à 8,3 (multiplet).

Analyse :
Calculée : C% 60,98 H% 3,51 Br% 25,36
Trouvée : C% 61,12 H% 3,35 Br% 25,34

### Exemple 2

Bromométhyl-8-phényl-2-4H-[1]-benzopyran-4-one (formule 1) $C_{16}H_{11}BrO_2$ PM = 315,17.

Dans un réacteur on place 880 ml de dioxane anhydre et 84,5 g (1,76 mole) d'hydrure de sodium. On porte le milieu à 80 °C et on ajoute goutte à goutte sous agitation un mélange de 277 g (1,32 mole) de méthoxyméthyl-3-méthoxy-2-benzoate de méthyle et 105,7 g (0,88 mole d'acétophénone. On poursuit

ensuite le chauffage à 80 °C pendant 3 heures. Après refroidissement on ajoute sous agitation 7 litres d'hexane. On laisse une nuit au repos. Le précipité formé est essoré, et est introduit par petites portions dans un mélange : — acide acétique : 1,3 litre, — eau : 2,6 litres, — benzène : 2,6 litres. La phase organique est décantée, lavée avec une solution de bicarbonate de sodium, séchée et le solvant est évaporé sous vide. On obtient 227,4 g d'une huile que l'on utilise sans purification dans l'étape suivante. Cette huile est solubilisée dans 911 ml d'acide acétique. On ajoute 650 ml d'acide bromhydrique à 62 % et le milieu est porté 3 heures à 80 °C. On refroidit à 40 °C et on verse sous agitation dans 12 litres d'eau. Le précipité formé est filtré, lavé à l'eau et recristallisé dans l'acétone. Poids obtenu : 123 g (Rdt : 51,3 %), $PF_G$ : 182-183 °C.

Exemple 3

Bromométhyl-8-(méthyl-4-phényl)-2-4H-[1]-benzopyran-4-one(formule 2) $C_{17}H_{13}BrO_2$. PM = 329,20.

Préparé selon l'exemple 2 à partir de 51,1 g (0,38 mole) de (méthyl-4-phényl)-1-éthanone, 120 g (0,571 mole) de méthoxy-2-méthoxyméthyl-3-benzoate de méthyle. Après isolement, l'huile obtenue est reprise par 348 ml d'acide acétique et 348 ml d'acide bromhydrique à 62 %. Poids obtenu : 53,3 g (Rdt : 41,8 %), $PF_K$ : 191 °C (Toluène), IR : µc = 0 (Pyrone) : 1 640 $cm^{-1}$. RMN (DMSO) δ en ppm par rapport au TMS, 3 H à 2,4 (singulet), 2 H à 5,1 (singulet), 1 H à 7 (singulet), 7 H de 7,3 à 8,3 (multiplet).

Exemple 4

Bromométhyl-8-(méthoxy-4-phényl)-2-4H-[1]-benzopyran-4-one (formule 3) $C_{17}H_{13}BrO_3$. PM = 345,20.

Préparé selon l'exemple 2 à partir de 57,14 g (0,38 mole) de (méthoxy-4-phényl)-1-éthanone, 120 g (0,571 mole) de méthoxy-2-méthoxyméthyl-3-benzoate de méthyle. Après isolement l'huile obtenue est reprise par 457 ml d'acide acétique et 325 ml d'acide bromhydrique à 62 %. Poids obtenu : 78 g (Rdt : 59,4 %), $PF_K$ : 165 °C (Toluène). IR : µc = 0 (pyrone) : 1 640 $cm^{-1}$. RMN ($CDCl_3$) en ppm par rapport au TMS, 3 H à 3,8 (singulet), 2 H à 4,8 (singulet), 1 H à 6,75 (singulet), 7 H de 7 à 8,3 multiplet.

Exemple 5

Bromométhyl-8-chloro-6-phényl-2-4H-[1]-benzopyran-4-one (formule 4) $C_{16}H_{10}BrClO_2$. PM = 349,62.

A partir de 69 g (0,32 mole) de (chloro-5-hydroxy-2-méthoxy-méthyl-3-phényl)-1-éthanone et 72,3 g (0,48 mole) de benzoate d'éthyle, la (chloro-5-hydroxy-2-méthoxyméthyl-3-phényl)-1-phényl-3-propanedione intermédiaire est isolée ($PF_K$ : 100 °C). Par traitement avec 300 ml d'acide acétique et 210 ml d'acide bromhydrique on obtient le produit attendu. Poids obtenu : 61,2 g (Rdt 54,7 %), $PF_K$ = 210 °C (Toluène, YR : µc : 0 (pyrone) : 1 640 $cm^{-1}$.. RMN ($CDCl_3$) en ppm par rapport au TMS, 2 H à 5 (singulet), 1 H à 7,1 (singulet), 7 H de 7,3 à 8,3 (multiplet).

Exemple 6

Bromométhyl-8-méthyl-6-phényl-2-4H-[1]-benzopyran-4-one (formule 5) $C_{17}H_{13}BrO_2$, PM : 329,20.

A partir de 204 g (1,05 mole) d'(hydroxy-2-méthoxyméthyl-3-méthyl-5-phényl)1-éthanone et 210 g (1,4 mole) de benzoate d'éthyle, après isolement l'huile obtenue est traitée par 520 ml d'acide acétique et 410 ml d'acide bromhydrique à 62 %. Poids obtenu : 108 g (Rdt : 31,2 %), $PF_K$ : 186 °C (acétone), IR : µc = 0 (pyrone) : 1 640 $cm^{-1}$. RMN (DMSO) δ en ppm par rapport au TMS, 3 H à 3,2 (singulet), 2 H à 5 (singulet), 1 H à 7 (singulet), 7 H de 7,3 à 8,3 (multiplet).

Exemple 7

Bromométhyl-8-(thényl-2)-2-4H-[1]-benzopyran-4-one (formule 6), $C_{14}H_9Br_2$, PM = 321,2.

A partir de 118 g (0,65 mole) d'hydroxy-2-méthoxyméthyl-3-phényl)-1 éthanone et 127 g (0,82 mole) d'α-thénoate d'éthyle, l'huile brute obtenue est traitée par 450 ml d'acide bromhydrique à 62 % et 600 ml d'acide acétique, selon l'exemple 4, on obtient 80,1 g (Rdt : 40 %) d'un solide. PF : 174 °C (éthanol). IR : µc = 0 (pyrone) : 1 640 $cm^{-1}$. RMN ($CDCl_3$) en ppm par rapport au TMS. 2 H à 4,8 (singulet), 1 H à 6,7 (singulet), 6 H de 7,1 à 8,3 (multiplet).

Exemple 8

(Bromométhyl)-8-(méthoxy-3-phényl)-2-4H-[1]-benzopyrancne-4 (formule 7) $C_{17}H_{13}BrO_3$. PM = 345,20.

Préparé selon l'exemple 2 à partir de 57,1 g (0,38 mole) de (méthoxy-3-phényl)-1-éthanone, et de 120 g (0.571 mole) de méthoxy-2-méthoxy-méthyl-3-benzoate de méthyle. Poids obtenu : 49,8 g (Rdt : 38 %), $PF_K$ = 160 °C (toluène-hexane). IR : µc = 0 = 1 655 $cm^{-1}$. RMN ($CDCl_3$), 3 H à 3,95 (singulet), 2 H à 4,8

(singulet), 1 H à 6,8 (singulet), 6 H de 7,0 à 7,9 (multiplet), 1 H à 8,2 (doublet de doublet, $J_1$ = 8 Hz ; $J_2$ = 2 Hz).

Exemple 9

Hydroxy-3-méthyl-8-phényl-2-4H-[1]-benzopyranone-4 (formule 8) $C_{16}H_{12}O_3$. PM = 252,27.

A une solution de 90,1 g (0,6 mole) d'(hydroxy-2-méthyl-3-phényl)-1-éthanone et de 63,7 g (0,6 mole) de benzaldéhyde dans 1,2 litre d'éthanone, on ajoute à température inférieure à 30 °C une solution de 81,6 g (2,04 moles) de pastilles de soude dans 163 ml d'eau. On agite une nuit à température ambiante, puis on ajoute 204 ml d'une solution de soude aqueuse à 20 %, dilués avec 4 l d'éthanol. On refroidit au bain de glace et on ajoute rapidement 825 ml d'une solution d'eau oxygénée à 15 %. On agite 4 heures à 25 °C puis on acidifie avec $H_2SO_4$ 30 % et on verse sur un mélange eau-glace (15 l). On laisse reposer une nuit, on filtre le précipité obtenu que l'on recristallise dans le toluène. Poids obtenu : 18,3 g (Rdt : 12 %). $PF_K$ = 180 °C. IR : µOH = 3 300 $cm^{-1}$, µc = 0 (pyrone) = 1 625 $cm^{-1}$ (épaulement à 1 640 $cm^{-1}$) RMN ($CDCL_3$), 3 H à 2,66 (singulet), 1 H à 6,7 (échangeable avec $D_2O$), 5 H de 7,2 à 7,7 (multiplet), 3 H de 8,0 à 8,5 (multiplet).

Exemple 10

Méthoxy-3-méthyl-8-phényl-2-4H-[1]-benzopyranone-4 (formule 9) $C_{17}H_{14}O_3$. PM = 266,30.

On chauffe à reflux pendant 16 heures un mélange de 18 g (0,071 mole) d'hydroxy-3-méthyl-8-phényl-2-4H-[1]-benzopyranone-4, 9,86 g (0,071 mole) de carbonate de potassium, 100 ml d'acétone et 9,9 g (0,078 mole) de diméthylsulfate. Puis on filtre à chaud, on refroidit le filtrat, on filtre le précipité obtenu que l'on recristallise dans l'acétone. Poids obtenu : 14,5 g (Rdt : 76 %). $PF_K$ = 143 °C. IR : µc = 0 (pyrone) = 1 645 $cm^{-1}$. RMN ($CDCL_3$), 3 H à 2,6 (singulet), 3 H à 3,97 (singulet), 5 H de 7,1 à 7,7 (multiplet), 3 H de 7,9 à 8,4 (multiplet).

Exemple 11

(Bromométhyl)-8-méthoxy-3-phényl-2-4H-[1]-benzopyranone-4 (formule 10) $C_{17}H_{13}BrO_3$. PM = 345,20.

On chauffe à reflux pendant 8 heures sous irradiation ultra-violette un mélange de 11 g (0,041 mole) de méthoxy-3-méthyl-8-phényl-2-4H-[1]-benzopyranone-4, 8,03 g (0,045 mole) de N-bromosuccinimide, 350 ml de tétrachlorure de carbone et 0,2 d'azobis-isobutyronitrile. On filtre à chaud, et on laisse le filtrat une nuit au réfrigérateur. On filtre, lave à l'eau et recristallise dans l'acétate d'éthyle. Poids obtenu : 6,2 g (Rdt : 43 %). $PF_K$ = 157 °C. IR : µc = 0 : 1 630 $cm^{-1}$. RMN ($CDCl_3$) 3 H à 3,95 (singulet), 2 H à 4,83 (singulet), 5 H de 7,2 à 7,9 (multiplet), 3 H de 8,1 à 3,5 (multiplet).

Exemple 12

Bromométhyl-8-méthoxy-6-phényl-2-4H-[1]-benzopyranone-4 (formule 11) $C_{17}H_{13}BrO_3$. PM = 345,20.

Préparé selon l'exemple 1 à partir de l'(hydroxy-2-méthoxy-5-méthoxy-méthyl-3-phényl)-1-phényl-3-propanedione-1,3 obtenue ci-dessus. Poids obtenu : 112,8 g (Rdt global : 60 %). $PF_K$ = 194 °C. IR : µc = 0 (pyrone) = 1 650 $cm^{-1}$. RMN (DMSO), 3 H à 3,9 (singulet), 2 H à 5,1 (singulet), 8 H de 7,0 à 8,3 (multiplet).

Exemple 13

(Bromométhyl)-8-(diméthoxy-3,4-phényl)-2-4H-[1]-benzopyranone-4 (formule 12). $C_{18}H_{15}BrO_4$. PM = 375,23.

Préparé selon l'exemple 2 à partir de 68,6 g (0,38 mole) de (diméthoxy-3,4-phényl)-1-éthanone et de 120 g (0,571 mole) de méthoxy-2-méthoxy-méthyl-3-benzoate de méthyle. Poids obtenu : 82 g (Rdt : 57 %). $PF_K$ = 185 °C (toluène-hexane). IR : µc = 0 (pyrone) = 1 630 $cm^{-1}$. RMN ($CDCl_3$), 3 H à 3,97 (singulet), 3 H à 4,0 (singulet), 2 H à 4,8 (singulet), 1 H à 6,8 (singulet), 5 H de 6,9 à 7,9 (multiplet), 1 H à 8,22 (doublet de doublet, $J_1$ = 8 Hz ; $J_2$ = 2 Hz).

Exemple 14

Diphényl-2,3-méthyl-8-4H-[1]-benzopyranone-4 (formule 13). $C_{22}H_{16}O_2$. PM = 312,37.

On chauffe 7 h entre 170 et 180 °C un mélange de 98 g (0,433 mole) d'(hydroxy-2-méthyl-3-phényl)-1-phényl-2-éthanone, 98 g (0,433 mole) d'anhydride benzoïque et 62,4 g (0,433 mole) de benzoate de sodium. On refroidit, reprend au benzène, lave à l'eau, puis avec une solution aqueuse de bicarbonate de sodium, puis à l'eau. On évapore le benzène sous vide et on recristallise le produit obtenu dans le toluène. Poids obtenu : 36,4 g (Rdt : 27 %). $PF_K$ = 209-210 °C. IR : µc = 0 = 1 630 $cm^{-1}$. RMN ($CDCl_3$) 3 H à 2,55

(singulet), 12 H de 7,1 à 7,7 (multiplet), 1 H à 8,15 (doublet de doublet, $J_1$ = 8 Hz ; $J_2$ = 2 Hz).

Exemple 15

Bromométhyl-8-diphényl-2,3-4H-[1]-benzopyranone-4 (formule 14) $C_{22}H_{15}BrO_2$. PM = 391,27.

Préparé selon l'exemple 13 à partir de 36 g (0,115 mole) de diphényl-2,3-méthyl-8-4H-[1]-benzopyranone-4. Après filtration de la succinimide, le filtrat est évaporé sous vide, le solide obtenu est repris à l'eau et filtré. Poids obtenu : 45 g (Rdt quantitatif). $PF_K$ = 143-147 °C. IR : μc = 0 (pyrone) = 1 635 $cm^{-1}$. RMN ($CDCl_3$), 2 H à 4,8 (singulet), 12 H de 7 à 7,9 (multiplet), 1 H à 8,25 (doublet de doublet, $J_1$ = 8 Hz ; $J_2$ = 2 Hz).

Exemple 16

Bromométhyl-8-(naphtyl-2)-2-4H-[1]-benzopyranone-4 (formule 15) $C_{20}H_{13}BrO_3$. PM = 362,22.

Préparé selon l'exemple 2 à partir de 105,1 g (0,5 mole) de méthoxy-2-méthoxyméthyl-3-benzoate de méthyle et 85,1 g (0,5 mole) de (naphthyl-2)-1-éthanone. Après isolement l'huile brute obtenue est reprise par 600 ml d'acide acétique et 426 ml d'acide bromhydrique à 62 %.

Par traitement selon la méthode habituelle on obtient 61 g (Rdt : 30 %) $PF_G$ = 190-192 °C (toluène). IR : μc = 0 (pyrone) = 1 650 $cm^{-1}$.

Exemple 17

Bromométhyl-8-(furyl-2)-2-4H-[1]-benzopyranone-4 (formule 16) $C_{14}H_9BrO_3$. PM = 305,13.

Préparé selon l'exemple 2 à partir de 105,1 g (0,5 mole) de méthoxy-2-méthoxyméthyl-3-benzoate de méthyle et 55 g (0,5 mole) de (furyl-2)-1-éthanone. Après isolement de l'huile brute, celle-ci est reprise par 600 ml d'acide acétique et 426 ml d'acide bromhydrique à 62 %. Par traitement selon la méthode habituelle on obtient 23,4 g (Rdt : 15 %). $PF_K$ = 210 °C. IR : μc = 0 (pyrone) = 1 660 $cm^{-1}$.

Exemple 18

Bromométhyl-8-cyclobexyl-2-4H-[1]-benzopyranone-4 (formule 17) $C_{16}H_{17}BrO_2$. PM = 321,22.

Préparé selon l'exemple 1 à partir de 18 g (0,062 mole) de cyclohexyl-1-(hydroxy-2-méthoxyméthyl-3-phényl)-3-propanedione-1,3. Poids obtenu : 15,2 g (Rdt : 76 %). $PF_K$ = 130 °C (acétone) — IR : μc = 0 (pyrone) = 1 645 $cm^{-1}$. RMN ($CDCl_3$), 11 H de 1,1 à 3,0 (multiplet), 2 H à 4,7 (singulet), 1 H à 6,2 (singulet), 1 H à 7,3 (triplet, J = 8 Hz), 1 H à 7,65 (doublet de doublet, $J_1$ = 8 Hz, $J_2$ = 2 $H_2$), 1 H à 8,15 (doublet de doublet, $J_1$ = 8 Hz, $J_2$ = 2 Hz).

Exemple 19

Méthyl-8-phényl-3-4H-[1]-benzopyranone-4 (formule 18) $C_{16}H_{12}O_2$. PM = 236,27.

On chauffe 8 heures à reflux un mélange de 85 g (0,375 mole) d'(hydroxy-2-méthyl-3-phényl)-1-phényl-2-éthanone, 500 ml de pyridine, 50 ml de pipéridine et 810 ml d'orthoformiate de triéthyle. Puis on verse dans un mélange d'acide chlorhydrique 6 N et de glace (3 L). On filtre le précipité obtenu et on recristallise dans l'isopropanol. Poids obtenu : 80,9 g (Rdt : 91 %). $PF_K$ = 110-111 °C. IR : μc = 0 = 1 650 $cm^{-1}$. RMN ($CDCl_3$), 3 H à 2,5 (singulet), 7 H de 7,1 à 7,8 (multiplet), 1 H à 8,02 (singulet), 1 H à 2,2 (doublet de doublet, $J_1$ = 8 Hz, $J_2$ = 2 Hz).

Exemple 20

Bromométhyl-8-phényl-3-4H-[1]-benzopyranone-4 (formule 19) $C_{16}H_{11}BrO_2$. PM = 315,17.

Préparé selon l'exemple 13 à partir de 80,9 g (0,34 mole) de méthyl-8-phényl-3-4H-[1]-benzopyranone-4 et de 73,4 g (0,41 mole) de N-bromosuccinimide. Poids obtenu : 88,2 g (Rdt : 82 %). $PF_K$ = 142 °C (acétat d'éthyle). IR : μc 0 (pyrone) = 1 640 $cm^{-1}$. RMN ($CDCl_3$), 2 H à 4,8 (singulet), 7 H de 7,2 à 8,0 (multiplet), 1 H à 8,18 (singulet), 1 H à 8,4 (doublet de doublet, $J_1$ = 8 Hz, $J_2$ = 2 Hz).

Exemple 21

Bromométhyl-8-méthyl-2-4H-[1]-benzopyranone-4 (formule 20) $C_{11}H_9BrO_2$. PM = 253,10.

Préparé selon l'exemple 1 à partir de 27,4 g (0,116 mole) de (méthoxy-2-méthoxyméthyl-3-phényl)-1-butanedione-1,3. Poids obtenu : 18 g. (Rdt : 61 %). $PF_K$ = 126-128 °C (cyclohexane - acétate d'éthyle), IR : μc = 0 = 1 670 $cm^{-1}$. RMN ($CDCl_3$), 3 H à 2,45 (singulet), 2 H à 4,73 (singulet), 1 H à 6,27 (singulet), 1 H de 7,1 à 7,5 (multiplet), 1 H à 7,77 (doublet de doublet, $J_1$ = 8 Hz, $J_2$ = 2 Hz), 1 H à 8,2 (doublet de doublet, $J_1$ = 8 Hz, $J_2$ = 2 Hz).

Exemple 22

Bromométhyl-8-(phénylméthyl)-2-4H-[1]-benzopyranone-4 (formule 21) $C_{17}H_{13}BrO_2$. PM = 329,20.

Préparé selon l'exemple 1 à partir de l'(hydroxy-2-méthoxyméthyl-3-phényl)-1-phényl-4-butanedione-1,3 préparée dans l'exemple précédent. Poids obtenu : 47,5 g (Rdt : 72 %). $PF_K$ = 128 °C (toluène-hexane). IR : μc = 0 (pyrone) = 1 670 $cm^{-1}$. RMN ($CDCl_3$), 2 H à 4,0 (singulet), 2 H à 4,63 (singulet), 1 H à 6,23 (singulet), 6 H de 7,1 à 7,55 (multiplet), 1 H à 7,7 (doublet de doublet, $J_1$ = 8 Hz, $J_2$ = 2 Hz), 1 H à 8,2 (doublet de doublet, $J_1$ = 8 Hz, $J_2$ = 2 Hz).

**Revendications** (pour les Etats contractants : BE, CH, DE, FR, GB, IT, LI, LU, NL, SE)

1. Bromoalkyl-8-4H-[1] benzopyran-4-ones, caractérisées par la formule

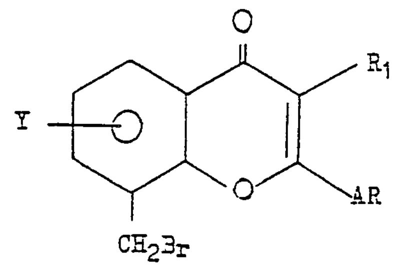

dans laquelle AR est un radical méthyle, phényle, méthylphényle, méthoxyphényle, diméthoxyphényle, thényle, furyle, naphtyle, cyclohexyle ou benzyle, $R_1$ est l'hydrogène, Y est l'hydrogène.

2. Bromoalkyl-8-4H-[1] benzopyran-4-ones, caractérisées par la formule

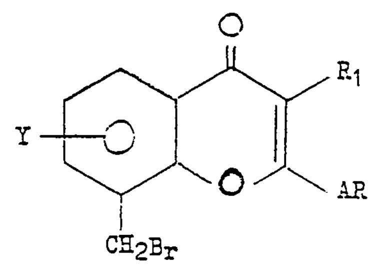

dans laquelle AR est un radical phényle, $R_1$ est l'hydrogène, Y est un radical méthyle ou méthoxy.

3. Bromoalkyl-8-4H-[1] benzopyran-4-ones, caractérisées par la formule

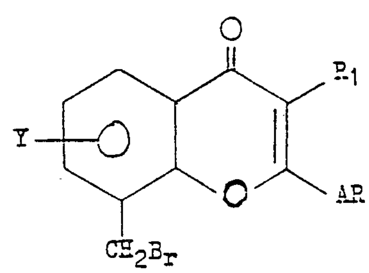

dans laquelle AR est un radical phényle ou hydrogène, $R_1$ est un radical phényle et Y est l'hydrogène.

4. Procédé de préparation des bromoalkyl-8-4H-[1] benzopyran-4-ones selon une quelconque des revendications 1 à 3, caractérisé par la cyclisation des propanediones-1,3 de formule

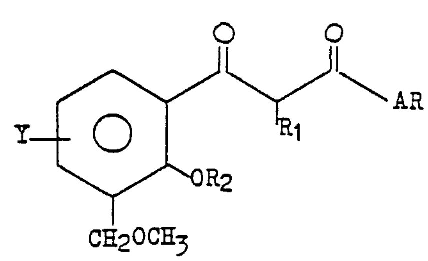

dans laquelle AR, $R_1$ et Y ont les mêmes significations que précédemment, $R_2$ est l'hydrogène ou un radical méthyle, en présence d'acide bromhydrique.

5. Application des composés intermédiaires selon une quelconque des revendications 1 à 3 dans la synthèse des produits pharmaceutiques et de pesticides.

**Revendications** (pour l'Etat contractant AT)

1. Procédé de préparation des bromoalkyl-8-4H-[1] benzopyran-4-ones représentés par la formule

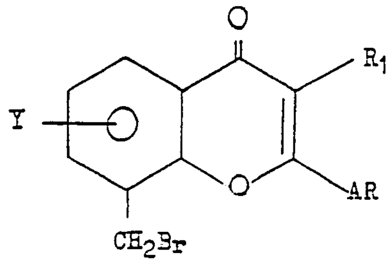

dans laquelle AR est un radical méthyle, phényle, méthylphényle, méthoxyphényle, diméthoxyphényle, thényle, furyle, naphtyle, cyclohexyle ou benzyle, $R_1$ est l'hydrogène et Y est l'hydrogène, caractérisé par la cyclisation des propanediones-1,3 de formule

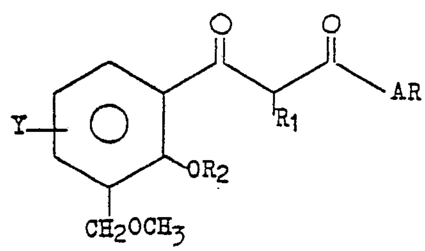

dans laquelle AR, $R_1$ et Y ont les mêmes significations que précédemment, $R_2$ est l'hydrogène ou un radical méthyle, en présence d'acide bromhydrique.

2. Procédé de préparation des bromoalkyl-8-4H-[1] benzopyran-4-ones représentés par la formule

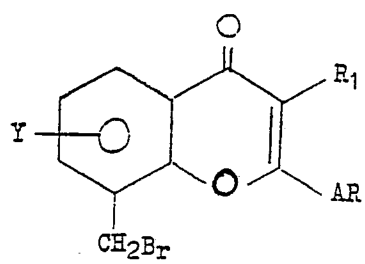

dans laquelle AR est un radical phényle, $R_1$ est l'hydrogène, Y est un radical méthyl ou méthoxy, caractérisé par la cyclisation des propanediones-1,3 de formule

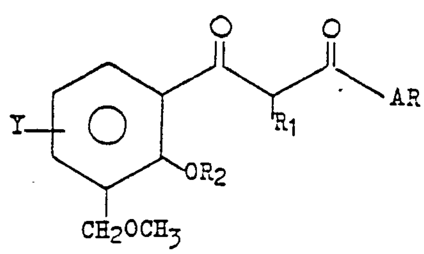

dans laquelle AR, $R_1$ et Y ont les mêmes significations que précédemment, $R_2$ est l'hydrogène ou un

radical méthyle, en présence d'acide bromhydrique.

3. Procédé de préparation des bromoalkyl-8-4H-[1] benzsopyran-4-ones caractérisées par la formule

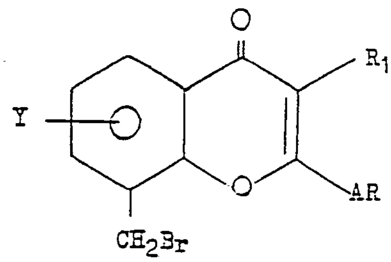

dans laquelle AR est un radical phényle ou hydrogène, $R_1$ est un radical phényle et Y est l'hydrogène, caractérisé par la cyclisation des propanediones-1,3 de formule

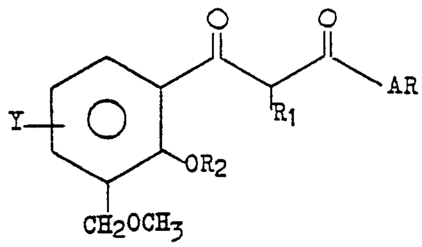

dans laquelle AR, $R_1$ et Y ont les mêmes significations que précédemment, $R_2$ est l'hydrogène ou un radical méthyle, en présence d'acide bromhydrique.

**Claims** (for the Contracting States : BE, CH, DE, FR, GB, IT, LI, LU, NL, SE)

1. Bromoalkyl-8-4H-[1]-benzopyran-4-ones, characterised by the formula

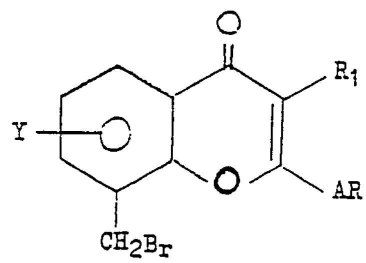

where AR is a methyl, phenyl, methylphenyl, methoxyphenyl, dimethoxyphenyl, thenyl, furyl, napthyl, cyclohexyl or benzyl radical, $R_1$ is hydrogen, Y is hydrogen.

2. Bromoalkyl-8-4H-[1]-benzopyran-4-ones, characterised by the formula

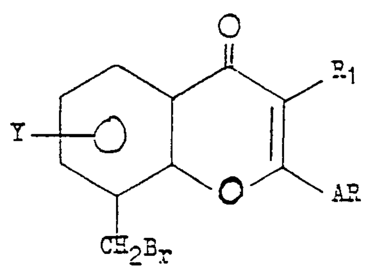

where AR is a phenyl radical, $R_1$ is hydrogen, Y is a methyl or methoxy radical.

3. Bromoalkyl-8-4H-[1]-benzopyran-4-ones, characterised by the formula

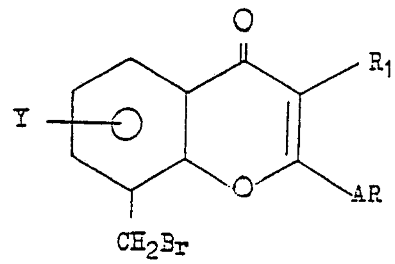

where AR is a phenyl or hydrogen radical, $R_1$ is a phenyl radical and Y is hydrogen.

4. Process for preparing bromoalkyl-8-4H-[1]-benzopyran-4-ones according to one of claims 1 to 3, characterised by the ring closure of the 1,3-propanediones of the formula

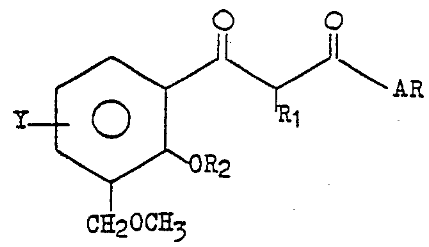

where AR, $R_1$ and Y have the same meanings as previously, $R_2$ is hydrogen or a methyl radical, in the presence of bromhydric acid.

5. Application of intermediate compounds according to one of claims 1 to 3 in the synthesis of pharmaceutical products and pesticides.

**Claims** (for the Contracting State AT)

1. Process for preparing bromoalkyl-8-4H-[1]-benzopyran-4-ones represented by the formula

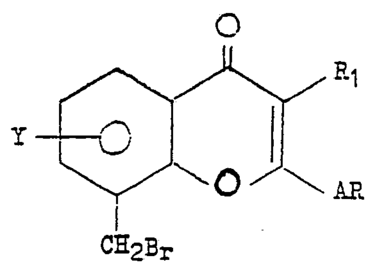

where AR is a methyl, phenyl, methylphenyl, methoxyphenyl, dimethoxyphenyl, thenyl, furyl, naphthyl, cyclohexyl or benzyl radical, $R_1$ is hydrogen and Y is hydrogen, characterised by the ring closure of the 1,3-propanediones of the formula

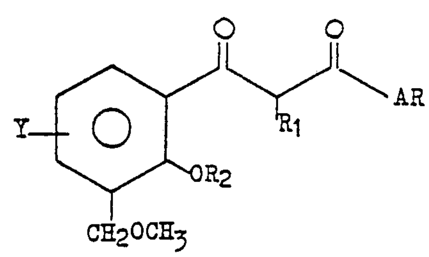

where AR, $R_1$ and Y have the same meanings as previously, $R_2$ is hydrogen or a methyl radical, in the presence of bromhydric acid.

2. Process for preparing bromoalkyl-8-4H-[1]-benzopyran-4-ones represented by the formula

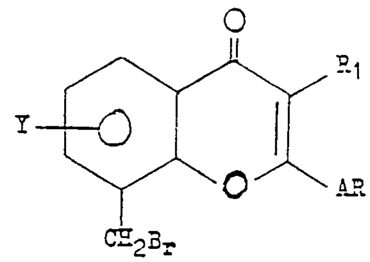

where AR is a phenyl radical, $R_1$ is hydrogen, Y is a methyl or methoxy radical, characterised by the ring closure of the 1,3-propanediones of the formula

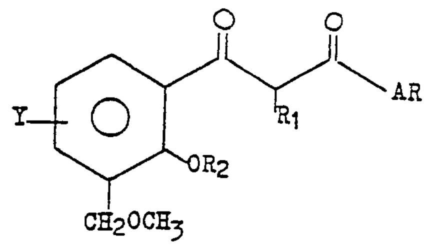

where AR, $R_1$ and Y have the same meanings as previously, $R_2$ is hydrogen or a methyl radical, in the presence of bromhydric acid.

3. Process for preparing bromoalkyl-8-4H-[1]-benzopyran-4-ones, characterised by the formula

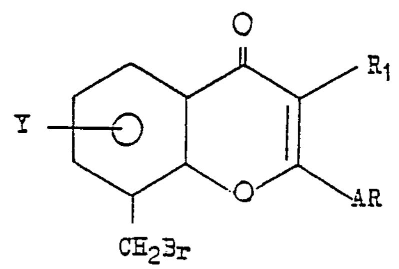

where AR is a phenyl or hydrogen radical, $R_1$ is a phenyl radical and Y is hydrogen, characterised by the ring closure of 1,3-propanediones of the formula

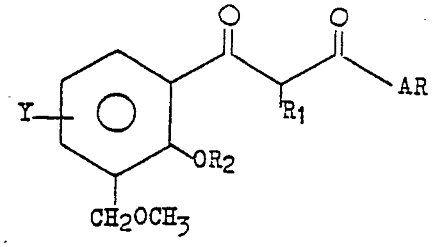

where AR, $R_1$ and Y have the same meanings as previously, $R_2$ is hydrogen or a methyl radical, in the presence of bromhydric acid.

**Patentansprüche** (für die Vertragsstaaten : BE, CH, DE, FR, GB, IT, LI, LU, NL, SE)

1. Bromoalkyl-8-4H-[1]-benzopyran-4-one, gekennzeichnet durch die Formel

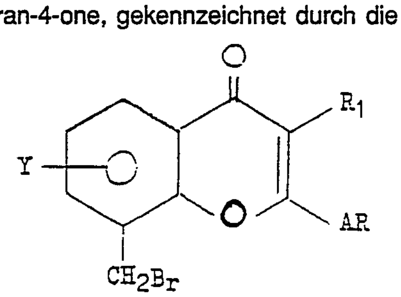

worin AR ein Methyl-, Phenyl-, Methylphenyl-, Methoxyphenyl-, Dimethoxyphenyl-, Thenyl-, Furyl-, Naphthyl-, Cyclohexyl- oder Benzylradikal, $R_1$ Wasserstoff, Y Wasserstoff ist.

2. Bromoalkyl-8-4H-[1]-benzopyran-4-one, gekennzeichnet durch die Formel

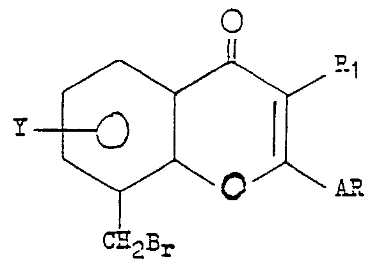

worin ein Phenylradikal, $R_1$ Wasserstoff, Y ein Methyl- oder Methoxyradikal ist.

3. Bromoalkyl-8-4H-[1]-benzopyran-4-one, gekennzeichnet durch die Formel

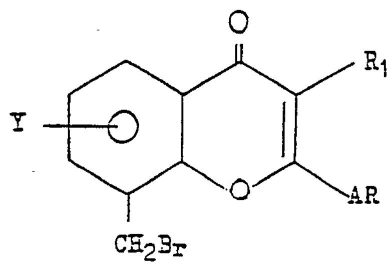

worin AR ein Phenylradikal oder Wasserstoff, $R_1$ ein Phenylradikal und Y Wasserstoff ist.

4. Verfahren zur Herstellung von Bromoalkyl-8-4H-[1]-benzopyran-4-onen nach einem der Ansprüche 1 bis 3, gekennzeichnet durch die Zyklisierung der Propandione-1,3 der Formel

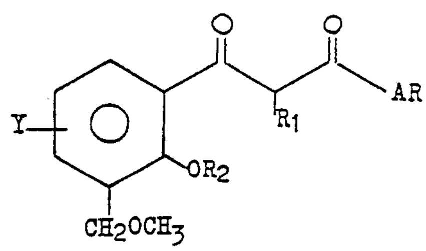

worin AR, $R_1$ und Y die oben angegebene Bedeutung haben, $R_2$ Wasserstoff oder ein Methylradikal ist, in Gegenwart von Bromwasserstoffsäure.

5. Verwendung der Zwischenverbindungen nach einem der Ansprüche 1 bis 3 für die Synthese von pharmazeutischen Produkten und Pestiziden.

**Patentansprüche** (für den Vertragsstaat AT)

1. Verfahren zur Herstellung der Bromoalkyl-8-4H-[1]-benzopyran-4-one, dargestellt durch die Formel

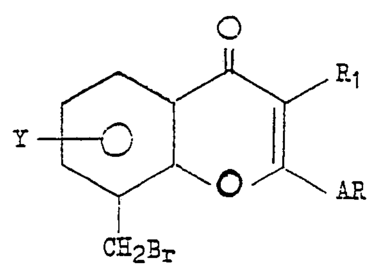

worin AR ein Methyl-, Phenyl-, Methylphenyl-, Methoxyphenyl-, Dimethoxyphenyl-, Thenyl-, Furyl-,

Napthyl-, Cyclohexyl- oder Benzylradikal, $R_1$ Wasserstoff und Y Wasserstoff ist, gekennzeichnet durch die Zyklisierung der Propandione-1,3 der Formel

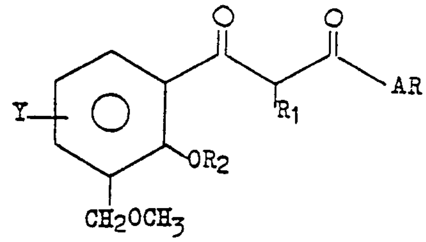

worin AR, $R_1$ und Y die oben angegebene Bedeutung haben, $R_2$ Wasserstoff oder ein Methylradikal ist, in Gegenwart von Bromwasserstoffsäure.

2. Verfahren zur Herstellung der Bromoalkyl-8-4H-[1]-benzopyran-4-one, dargestellt durch die Formel

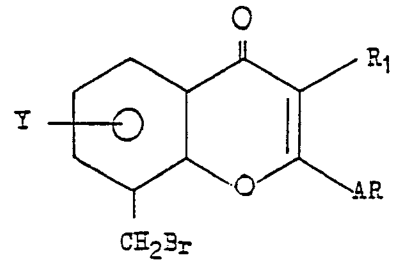

worin AH ein Phenylradikal, $R_1$ Wasserstoff, Y ein Methyl- oder Methoxyradikal ist, gekennzeichnet durch die Zyklisierung der Propandione-1,3 der Formel

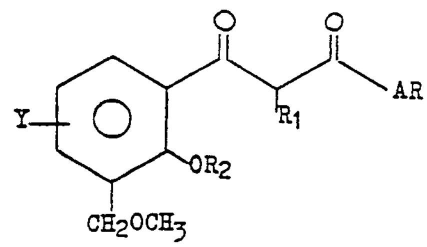

worin AR, $R_1$ und Y die oben angegebene Bedeutung haben, $R_2$ Wasserstoff oder ein Methylradikal ist, in Gegenwart von Bromwasserstoffsäure.

3. Verfahren zur Herstellung der Bromoalkyl-8-4H-[1]-benzopyran-4-one, dargestellt durch die Formel

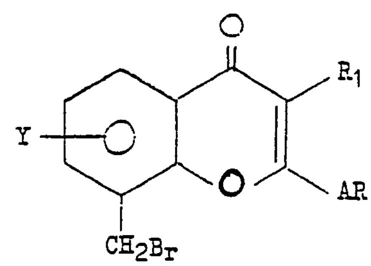

worin AR ein Phenylradikal oder Wasserstoff, $R_1$ ein Phenylradikal und Y Wasserstoff ist, gekennzeichnet durch die Zyklisierung der Propandione-1,3 der Formel

(Siehe Formel Seite 13 f.)

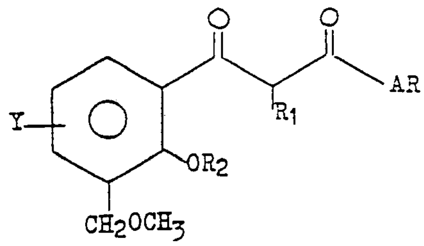

worin AR, $R_1$ und Y die oben angegebene Bedeutung haben, $R_2$ Wasserstoff oder ein Methylradikal ist, in Gegenwart von Bromwasserstoffsäure.

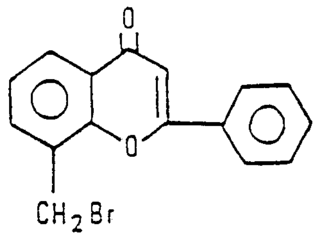

FORMULE 1

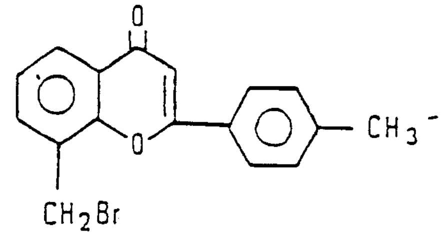

FORMULE 2

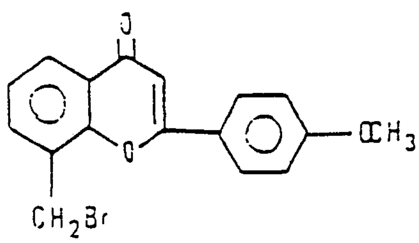

FORMULE 3

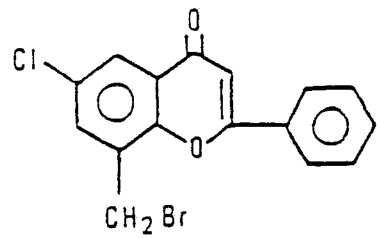

FORMULE 4

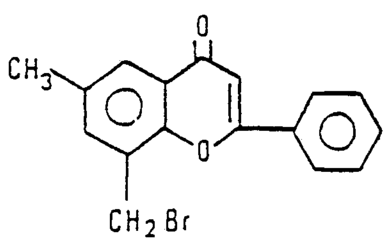

FORMULE 5

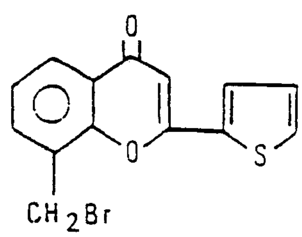

FORMULE 6

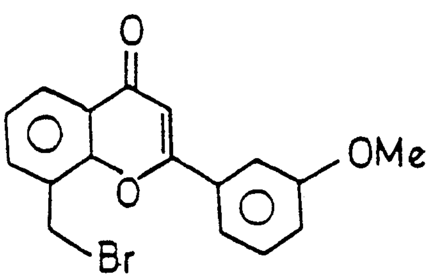

FORMULE 7

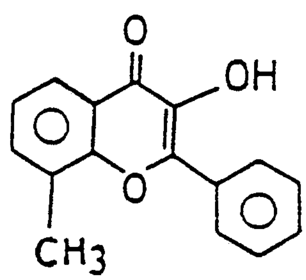

FORMULE 8

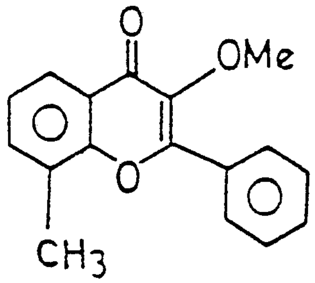

FORMULE 9

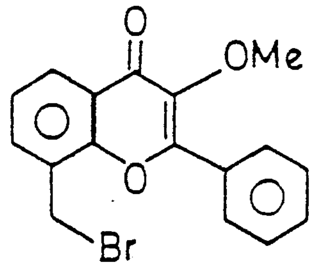

FORMULE 10

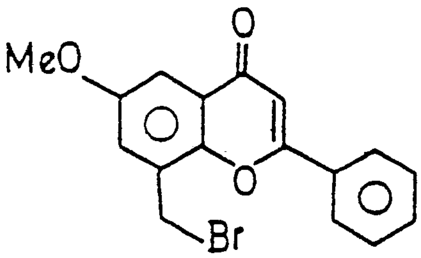

FORMULE 11

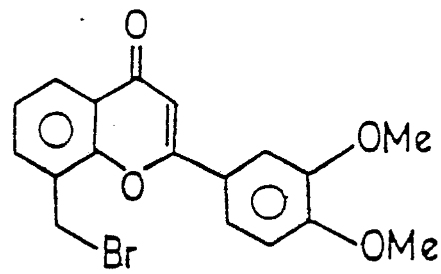

FORMULE 12

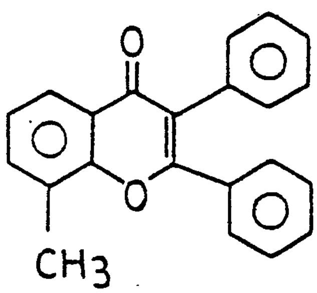

FORMULE 13

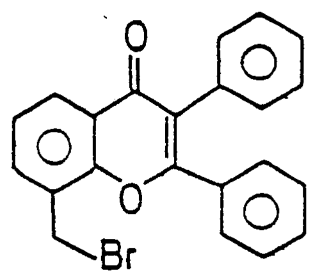

FORMULE 14

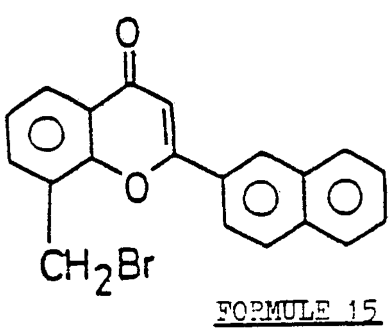

FORMULE 15

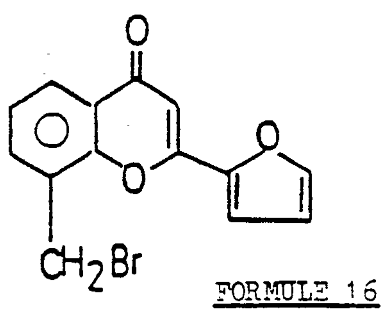

FORMULE 16

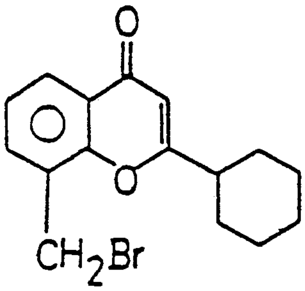

FORMULE 17

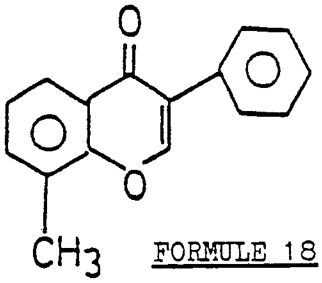

FORMULE 18

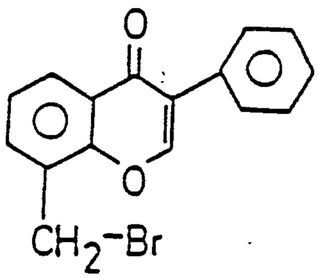

FORMULE 19

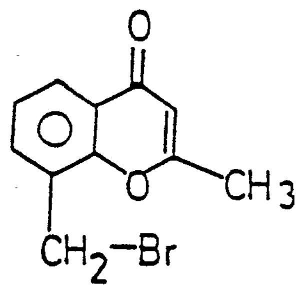

FORMULE 20

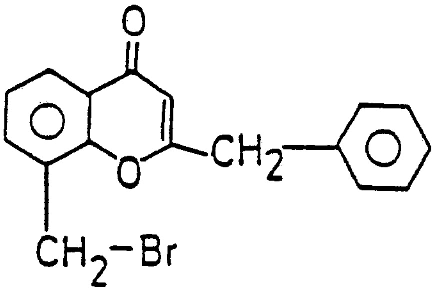

FORMULE 21